Europäisches Patentamt

European Patent Office

Office européen des brevets

(11) Publication number: **0 191 355**
**B1**

# EUROPEAN PATENT SPECIFICATION

(45) Date of publication of the patent specification: 19.04.89

(51) Int. Cl.⁴: **A 41 B  13/02,** C 09 J  7/04

(21) Application number: **86101095.7**

(22) Date of filling: **28.01.86**

(54) A fastener tape for disposable diaper.

(30) Priority: **30.01.85 JP 16281/85**
**09.03.85 JP 46733/85**
**09.03.85 JP 46734/85**
**13.03.85 JP 49654/85**

(43) Date of publication of application:
**20.08.86 Bulletin 86/34**

(45) Publication of the grant of the patent:
**19.04.89 Bulletin 89/16**

(84) Designated Contracting States:
**DE FR GB SE**

(56) References cited:
**EP-A-0 113 464**
**DE-A-2 517 790**
**FR-A-2 403 036**
**GB-A-1 226 841**
**US-A-4 051 853**

(73) Proprietor: **Kao Corporation, 14- 10, Nihonbashi Kayabacho 1-chome, Chuo- Ku Tokyo 103 (JP)**

(72) Inventor: **Ito, Osamu, 6-5- 1 Motoimaizumi, Utsunomiya- shi Tochigi- ken (JP)**
Inventor: **Toyoda, Harumitsu, 117 Hoedo- machi, Utsunomiya- shi Tochigi- ken (JP)**

(74) Representative: **Patentanwälte Grünecker, Kinkeldey, Stockmair & Partner, Maximilianstrasse 58, D-8000 München 22 (DE)**

EP 0 191 355 B1

**Description**

(1) Field of the Invention

The present invention relates to a fastener tape for disposable diaper, so improved as to facilitate putting on of disposable diaper, to reduce a discomfortable oppression exerted around a wearer's waist during use thereof and to improve fitment therearound.

(2) Description of the prior Art

It is vell known to use pressure-sensitive adhesive tape as means to fasten disposable diaper on the wearer's body. As such pressure-sensitive adhesive tape, various types have already been proposed. For example, Japanese patent publication 5 234 978 discloses pressure-sensitive adhesive tape without use of release paper and Unexamined Disclosure of Japanese Utility Model Application 5 575 608 describes three-layered Pressure-sensitive adhesive tape adapted to be repeatedly used. These fastener tapes of prior art have been adopted by most of disposable diapers on the market. However, these pressure-sensitive adhesive tapes serve only to fasten the respective disposable diapers on the wearers' bodies and no information on characteristics or properties of base tapes used in these fastener tapes of prior art can be found in said Publication and Disclosure.

As said base tape, paper, polyethylene- and polypropylene-film, non-woven fabric and woven fabric have generally be used independently or in combination. Any way, these materials have no elasticity.

Although still not commercially available, it has recently been proposed to make the base tape elastic so that the diaper may comfortably fit around the wearer's waist and thereby excretion may be effectively prevented from leakage. For example, Unexamined Disclosure of Japanese patent Application 5 168 345 describes fine elastic bodies such as rubber bands connected between fastener tapes so that said rubber bands fit around the wearer's waist. Unexamined Disclosure of Japanese Utility Model Application 5 7157 209 proposes a fastener tape centrally provided with elastic body so that the central portion of the fastener tape becomes elastic. However, these two Disclosures contain no description of the elastic bodies used therein. Accordingly, these elastic bodies seem to have a substantially perfect elasticity, i. e., to exhibit a substantially same stretch even after previously stretched so far as it is subjected to the same tension as the previous tension.

With the fastener tape utilizing the base tape having no elasticity, a position of the fastener tape is fixed once the disposable diaper has been put on the wearer, so that, if the diaper has been put on loosely, there will occur a gap between the diaper and the wearer, causing excretion leakage during use and, if the diaper has been put on tightly, there will occur an excessive oppression around the wearer's abdomen, back and legs with a danger that the wearer's skin will be injured in an extreme case.

With the fastener tape utilizing the elastic body having the substantially perfect elasticity as mentioned above as the base tape. an excessively low elasticity will result in that the fastener tape exhibits inconveniently excessive stretch during putting on of the disposable diaper while an excessively high elasticity will result in that the fastener tape exhibits inconveniently excessive contraction and therefore, discomfortable oppression around the wearer's waist. With the fastener tape adopting the base tape having practically perfect elasticity such as natural rubber or synthetic rubber, if the tape is too thin, a sufficient firmness to facilitate putting on of the disposable diaper cannot be obtained. If the base tape is made thicker to assure a desired firmness, there occurs the danger that the wearer's skin is injured as in the case of the non-elastic base tape and, in addition, a cost is increased. Furthermore, the fastener tape utilizing the base tape having the practically perfect elasticity and being excessively thin necessarily reduces productivity and increases the cost, since a coating speed of adhesive is extremely limited due to said poor firmness. During a secondary processing also, said poor firmness necessitates a reduction of productivity.

The fastener tape described in the previously mentioned Unexamined Disclosure of Japanese Utility Model Application 5 7157 209 has no firmness necessary for convenient use, since the elastic portion forming a part of the base tape is made of ordinary elastic sheet.

With the fastener tape described In the previously mentioned Unexamined Disclosure of Japanese patent Application 5 168 345, the wearer's skin is oppressed and sometimes becomes reddish, since the fine elastic bodies such as rubber bands directly oppress the wearer's waist.

**Summary of the invention**

An object of the present invention is to provide a novel and improved fastener tape for disposable diaper adapted to be conveniently put on a wearer, to moderate a possible oppression around the wearer's waist and to improve fitment of the disposable diaper around the wearer's waist.

Another object of the present invention is to provide a novel and improved fastener tape for disposable diaper being properly elastic, soft but firm and adapted to prevent an excretion leakage effectively.

Still another object of the present invention is to provide a novel and improved fastener tape for disposable diaper adapted to facilitate its manufacturing and processing.

These objects are achieved, in accordance with the present invention, by a fastener tape for disposable diaper comprising a base tape carrying thereon an adhesive layer, characterized in that said base tape has an elasticity that a hysteresis is generated between a tension exerted on said base tape and a stretch of said base tape: and a hysteresis loss ratio of a hysteresis curve lies vithin the menge og 20 to 80 %.

Said objects are achieved, in accordance with the present invention, by provision of a fastener tape characterized in that a hysterisis is genereted between a tensile force exerted on the third portion and a stretch of said third portion, and a hysterisis loss ratio of a hysterisis curve lies within comprising a base tape carrying thereon an adwhesive layer, said base tape consisting of a first portion and a second portion both being non-elastic and forming opposite ends of the base tape and an intermediate or third portion having an elasticity the range of 20 to 80 %.

The fastener tape according to the present invention will be described more in detail. Said base tape preferably presents an elasticity such that a stretch of 30 to 400 % is obtained under a tensile force of 1000 to 1500 g/cm. Excessively stretching would make it inconvenient to put the disposable diaper on the wearer and insufficiently stretching would make it difficult to obtain a suitable fitment of the diaper around the wearer's waist. To obtain an effective fitment of the diaper around the wearer's waist, said elastic body preferably presents a permanent strain of less than 30 % under a stretching of 100 %.

It is also desired that there is generated between the tension exerted on said base tape and the stretching thereof a hysteresis and, therefore, a hysteresis loss. Such base tape has a characteristic that a force occuring when the base tape contracts is less than a force necessary to stretch such base tape. In other words, such base tape exhibits the characteristic that there occurs a proper high drag when the fastener tape is under a tensile force to put the disposable diaper on the wearer while a relatively weak contracting force occurs once the diaper has been put on the wearer.

In the fastener tape according to the present invention, a hysteresis loss ratio of the base tape must lie within the range of 20 to 80 %, preferably 30 to 75 %. So long as the hysteresis loss ratio lies within this range, a proper high drag is generated to a tensile force when the fastener tape is stretched to put the disposable diaper on the wearer so that any excessive stretch of the fastener tape due to an excessive tensile force can be avoided while, once the disposable diaper has been put on the wearer, a properly weak contraction force maintains a suitble fitment around the wearer's waist so that any discomfortable oppression around the wearer's waist can be avoided. A hysteresis loss ratio less than 20 % would eliminate a desired difference between a tensile force for putting on of the diaper and a contractile force after wearing, resulting in the various problems encountered by the base tape of prior art. On the contrary, a hysteresis loss ratio more than 80 % would make the base tape substantially non-elastic and make it impossible to improve the fitment of the disposable diaper around the wearer's waist.

In one embodiment of the present invention, said base tape consists only of a base sheet having said hysteresis loss ratio.

Such base sheet is classified into two groups, i. e., a base sheet of well known construction using a specific elastic material as its base material and a base sheet of a specific construction having a desired elasticity regardless of base material.

Elastic bodies useful for the former base sheet include, for example, polyurethane, modified polyethylene, polyvinylalcohol and polybutadiene which are unoriented, polyvinylchloride, ethylene-propylenedienemixture, SBS (polystyrene-polybutadiene-polystyrene copolymer), SEBS [polystyrene-poly(ethylene-butylene)-polystylene block copolymer] and propylene rubber independently or in combination thereof, so far as it has a hysteresis loss ratio of 20 to 80 %.

As the latter base sheet, a base sheet is useful which has a plurality of weakened areas disposed adjacent to one another, each weakened area being elongate transversely of the sheet so that substantially no drag and stress develop to any deformation transversely of the sheet.

Now said base sheet having the weakened areas will be described in detail. The weakened area formed in said base sheet means the area which develops substantially no drag and stress to any deformation, in other words, the area which includes substantially no means for transmission of a force. Stress such as a tensile force exerted on the sheet, generally trends to deform this sheet and the sheet, in turn, develops drag and stress to this deformation. However, if this sheet is partially deprived of its means for transmission of force, i. e., said sheet is partially provided with areas in which said means for transmission of force are substantially absent, these areas practically exhibit no drag and stress to any deformation. Such areas are designated as the weakened areas in accordance with the present invention.

Said weakened areas are not limited with respect to their shapes and need not be uniform, so far as they are elongate transversely of the sheet. Examples of such weakened area elongate transversely of the sheet include elongate openings and slits such as elongate rhombic and rectangular ones formed in the base sheet. These openings and slits may be closed by film or the like, on condition that the weakened area still never develops a practical drag and a stress to any deformation transversely of the sheet. The base sheet provided with such weakened areas structurally presents an elastic deformation in response to a tensile force exerted laterally of the weakened area. i. e., longitudinally of the sheet. To generate an effective elasticity laterally of each weakened area i. e., longitudinally of the sheet, a length of the weakened area transversely of the sheet, depending on its particular shape, is preferably 1 to 6 mm and 1.3 times a length longitudinally of the sheet, particularly 1.5 times or higher. If the length of each weakened area transversely of the sheet is less than 1.3 times of its length longitudinally of the sheet, an elastic deformation occurring in the fastener tape according to the present invention would be insufficient to obtain an effective elasticity required for the disposable diaper. The number of these weakened areas elongate transversely of the sheet is preferably 4 to 120/cm$^2$, depending on their shapes.

Said weakened areas are formed elongate transversely of the fastener tape according to the present invention. Transverse direction of the fastener tape corresponds to vertical direction of the disposable diaper provided with the fastener tape according to the present invention when the diaper has been put on the wearer. The fastener tape of the invention thus expands and contracts transversely of the disposable diaper when the latter is put on, since the base sheet structurally presents an elastic deformation transversely of the weakened areas.

Said weakened areas elongate transversely of the sheet are provided in plurallity adjacent to one another. However, they are not continuous but with interposition of areas other than the weakened areas. The greater the number of these weakened areas extending transversely of the sheet and the larger the shift between each pair of adjacent weakened areas transversely of the sheet, the larger the elastic deformation exhibited by the base sheet. Although these weakened areas extending transversely of the sheet may take the form of openings or slits, the base sheet may be constructed as a net in which the respective meshes function as the weakened areas extending trasversely of the sheet to facilitate manufacturing and to improve wearing comfort. When the areas other than the weakened areas are defined by strands, a thickness of each strand is preferably 50 deniers or larger, particularly 150 to 15000 deniers to give the fastener tape according to the present invention an effective firmness. Furthermore, it is also possible that areas other than the weakened areas extending transversely of the sheet may be provided with other weakened areas which do not extend transversely of the sheet or are not elongate. In such case, said weakened areas which do not extend transversely of the sheet or are not elongate contribute in a different manner or do not contribute at all to the elastic deformation of the base sheet, depending on their shapes and orientations.

When the base sheet includes said weakened areas, the fastener tape according to the present invention is adequately elastically deformable, whether material of the base sheet is elastic or not, since the base sheet is structurally elastically deformable owing to said weakened areas, even when the base sheet material itself is not elastic. Therefore, no limitation is imposed on the base sheet material. Suitable materials for the base sheet include, for example, polyethylene, poly-, propylene, polyester, nylon, ethylene-vinyl acetate copolymer, modified polyethylene, and celluloses such as paper. As the base sheet material, non-elastic materials are rather preferable in view of the firmness. Weight per unit area of the base sheet is preferably 10 to 200g/m². 

Of said base sheets useful for the present invention, NETLON® available from Tokyo Polymer Co. is particularly preferable. This base sheet comprises a plurality of strands slightly oblique in opposite direction with respect to the width of the sheet, forming a net including a plurality of integrated intersecting points In the net formed by these strands, areas having no strands, i. e., meshes provide rhombic openings elongate transversely of the sheet, which function as the weakened areas.

Said base tape in the fastener tape according to the present invention may comprise said base sheet and an elastic material integrated therewith, instead of the single component structure as mentioned above. Such integration of the elastic material permits the base sheet as a whole to have its hysteresis loss ratio within said specified range, even when the base sheet material itself has a hysteresis loss ratio outside said specified range. The base sheet including the weakened areas as mentioned above will get its weakened areas covered with said elastic material. However, even after the weakened areas have been covered with the elastic material, the base sheet maint its firmness and usually keeps its softness also, so long as the elastic material is not thicker than the base sheet itself. Thus the elastic deformarbility of the weakened areas is never affected. Covering the weakened areas with the elastic material is rather advantageous in that coating or formation of the adhesive layer is facilitated.

Said elastic material may be applied in form of a sheet by means of heat, adhesive etc. on front or rear surface of the base sheet, or the base sheet may be impregnated or coated with resin dissolved in solvent, or monomer or oligomer itself, or dissolved in solvent, and/or may be polymerized or crosslinked using electron rays, ultraviolet rays, heat or like. Useful elastic materials include polyurethane, polybutadiene, ethylenevinylacetate copolymer, modified polyethylene, styrenebutadiene rubber, isotactic isoprene rubber, SBS (polystyrene-polybutadiene-polystyrene copolymer), SIS (polystyrene-polyisoprene-polystyrene copolymer), natural rubber, chloroprene rubber, nitril rubber, ethylene-propylene rubber, ethylene propylenediene mixture, butadiene rubber and any othe materials, so far as being elastic. When the elastic material is applied in form of a sheet upon the base sheet, the elastic material is used in form of film, non-woven fabric, woven fabric or the like. It is unnecessary that all of the weakened areas of the base sheet are covered with the elastic material. Furthermore, not only the weakened areas but also the remaining areas may be covered with the elastic material without reduction of the desired effect.

In the fastener tape according to the present invention, the base sheet need not necessarilly exhibit the characteristics as have been described above at every portion thereof. In consideration of ease for use and processing of the fastener tape, it is rather preferable that the base tape comprises first and second portions disposed on opposite ends and having no elasticity and an intermediate or a third portion as an elastic portion having said hysteresis loss ratio.

As a preferred embodiment of such base tape, an arrangement may be considered such that the base sheet comprises the first and second portion forming the opposite ends of the sheet as the non-elastic portions and the intermediate or the third portion including a plurality of weakened areas elongate transversely of the sheet and presenting substantially no drag and stress in response to any deformation transversely of the sheet.

Now said first and second portion will be described in detail. These portions must be nonelastic and this requirement may be satisfied in various manners. or example, these portions may be non-elastic sheets of well

4

known art merely connected by heat, adhesive or the like to the opposite ends of the third portion, or the base sheet or the base tape constituting the third portion may be prolonged in opposite directions and then said non-elastic sheets of well known art are applied by means of heat, adhesive or the like on front or rear surface of associated extensions of said base sheet or base tape, or the base sheet or base tape constituting the third portion may be impregnated o coated with resin dissolved in solvent, monomer or oligomer itself or dissolved in solvent and then subjected to polymerization and crosslinking using electron rays, ultraviolet rays, heat or the like to make these portion non-elastic. When the third portion comprises, as previously mention, the base sheet including the weakened areas elongate transversely of the sheet and the elastic material integrated therewith, said base tape may be prolonged in opposite directions and then these extensions of the elastic material may be crosslinked, polymerized or heat calender processed to obtain the first and second non-elastic portions.

The fastener tape according to the present invention comprises said base tape wholly or partially coated with the adhesive layer. Particularly when he base tape consists of, as previously mentioned, the first portion, the second portion and the third portion, all the surfaces of said three portions may be coated with the adhesive layer, but coating of the adhesive on the third portion which is soft and elastic is relatively diffucult and may often increase the manufacturing cost, so that it is preferred to coat wholly or partially the surfaces of the first portion and the second portion with the adhesive. Such procedure cause no problem in use of the fastener tape according to the present invention. Adhesive used to form the adhesive layer may be selected at will from various types such as solvent type, emulsion type, hot melt type and any other types.

The fastener tape according to the present invention is used, as in the conventional fastener tape, to connect the front body and the rear body of the disposable diaper at the waist level at opposite sides.

The fastener tape for disposable diaper according to the present invention, as aforementioned, includes the base tape having a proper elasticity and a hysteresis loss ratio of 20 to 80 % and thereby provides a remarkable effect such that the fastener tape can be easily handled to put the disposable diaper on the wearer, a possible oppression exerted around the wearer's waist can be reduced as effectively as possible, a fitment of the disposable diaper around the wearer's waist is improved and thereby any excretion leakage about the waist can be prevented.

When the base tape consists only of the base sheet including a plurality of the weakened areas extending transversely of the sheet adapted to develop substantially no drag and stress in response to a deformation transversely of the sheet, or comprises said base sheet and the elastic material integrated therewith, the fastener tape for disposable diaper according to the present invention provides another effect such that, even if the tape as a whole is made thicker, a proper softness is maintained, a firmness is relatively high with a same weight per unit area, the areas other than the weakened areas such as the strands may be thicker without a danger to injure the wearer's skin and rather facilitates wearing and use of the disposable diaper.

The fastener tape is substantially not deformed transversely of the base tape in response to any tensile force when the base tape comprises the base sheet including said weakened area, so that, in a secondary processing, the base tape may be fed transversely thereof under a tension in this direction without a substantial deformation. Accordingly, a mechanical speed may be increased during the secondary processing and a productivity is improved by performing the secondary processing of the fastener tape for disposable diaper according to the present invention in the manner as mentioned just above. Moreover, such effect is achieved in coating the base tape with adhesive.

When the opposite ends of the fastener tape for disposable diaper according to the present invention comprise the first portion and the second portion both being non-elastic, these end portions may be easily gripped and handled.

## Brief description of the drawings

The present invention will become more fully understood from the detailed description given hereinbelow and accompanying drawings which are given by way of illustration only, and thus not limitative of the present invention, and wherein:

Fig. 1 is a graphic diagram illustrating a hysteresis curve exhibited by base material of fastener tape according to the present invention;

Fig. 2 is a plan view illustrating a first embodiment of the present invention as attached to disposable diaper;

Fig. 3 is an enlarged section taken along a line X - X in Fig. 1;

Fig. 4 is an enlarged longitudinal section taken along an essential portion of the first embodiment;

Fig. 5 is a sectional view similar to Fig. 4 but illustrating a second embodiment of the present invention;

Fig. 6 is a plan view illustrating a third embodiment of the present invention as attached to disposable diaper at a waist level on one side;

Fig. 7 is a section taken along a line Y - Y in Fig. 6;

Fig. 8 is a longitudinal section similar to Fig. 4 but illustrating a fourth embodiment of the present invention;

Fig. 9 through 12 are enlarged plan views respectively showing essential portions of base sheets having weakened areas of patterns different from that as shown in Fig. 6;

Fig. 13 is a longitudinal section illustrating a fifth embodiment of the present invention as attached to disposable diaper at a waist level on one side;

Fig. 14 is a longitudinal section similar to Fig. 13 but illustrating a sixth embodiment of the present invention,

Fig. 15 is a longitudinal section similar to Fig. 13 but illustrating a seventh embodiment of the present invention;

Fig. 16 is a longitudinal section similar to Fig. 13 but illustrating a eighth embodiment of the present invention;

Fig. 17 is another longitudinal section of the embodiment as shown in Fig. 16;

Fig. 18 is an enlarged plan view illustrating an essential portion of the base sheet incorporated in the embodiment as shown by Fig. 16;

Fig. 19 is an enlarged plan view illustrating an essential portion of the base tape incorporated in the embodiment as shoen by Fig. 16;

Fig. 20 is a longitudinal section illustrating a ninth embodiment of the present invention: and

Fig. 21 is a longitudinal section illustrating a tenth embodiment of the present invention.

## Detailed description of the preferred embodiment

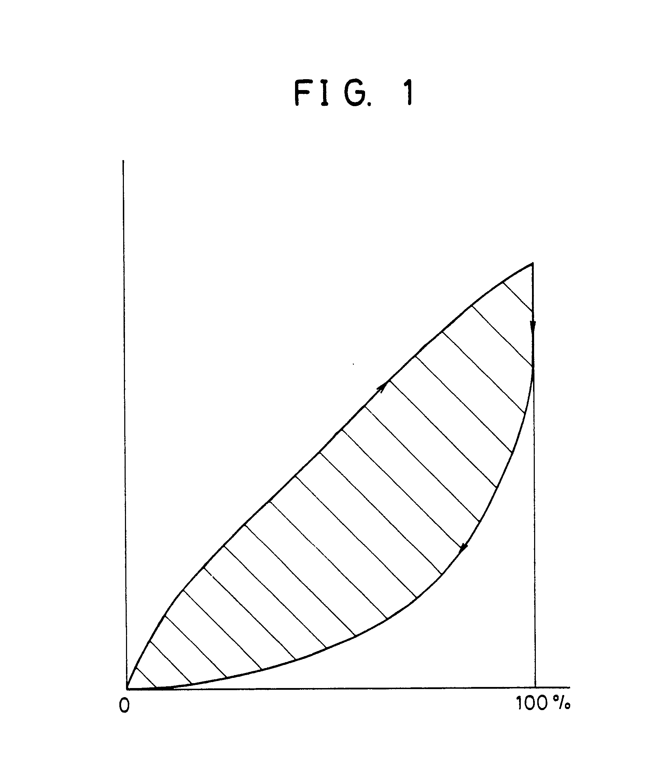

Fig. 1 is a graphic diagram illustrating a hysteresis curve exhibited by base material of fastener tape according to the present invention, wherein a hysteresis loss ratio can be calculated from;

$$[ S / (S + R) ] \times 100 \ (\%)$$

Where S represents an area defined by oblique lines as a hysteresis loss and R represents an area underlying S. It should be noted here that the hysteresis curve in Fig. 1 is based on a tensile test utilizing a tester TENSILON in which a sample of 25 mm wide base tape was stretched by 100 % under a tension of 100 mm/min. and then released. The arrows in Fig. 1 indicate a stress (tension) and a strain (elongation), respectively. The vertical axis in Fig. 1 indicates the stress, and the horizontal axis in Fig. 1 indicates the strain. As seen in Fig. 1, the tension during stretching (upper curve) is higher than the tension after put on a wearer (lower curve).

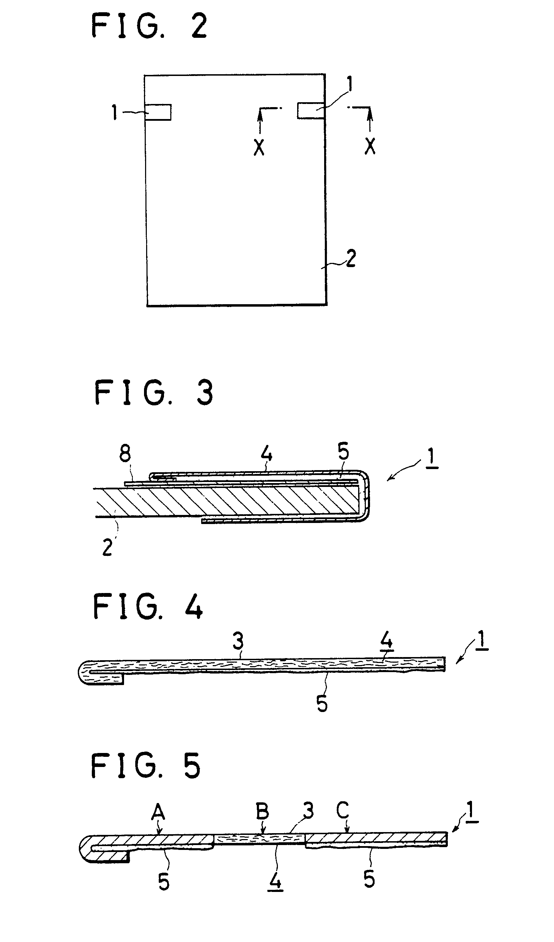

Figs. 2 and 3 are plan view and enlarged section taken along the line X - X, respectively, illustrating the first embodiment of the fastener tape according to the present invention as attached to the rear body of disposable diaper at the waist level on one side. Reference numeral 1 designates a fastener tape and reference numeral 2 designates disposable diaper. The fastener tape 1 comprises, as seen in Fig. 4 illustrating the essential portion thereof in an enlarged longitudinal section as prior to attachment, a base tape 4 consisting only of a base sheet 3 made of elastic material having a hysteresis loss ratio of 20 to 80 % and well known structure, and an adhesive layer 5 extending over an entire surface of said base tape 4. The fastener tape 1 is adhesively attached to disposable diaper 2 at the waist level on opposite sides. More specifically, the fastener tape 1 is attached to the rear body of disposable diaper 2 on outer and inner sides of said rear body with interposition of said adhesive layer 5. In acutual use of the fastener tape 1, a portion of the fastener tape 1 adhesively attached to the rear body of disposable diaper 2 at the waist level on opposite sides which is adhesively attached to the inner surface of the rear body is released and the portion thus released is then adhesively attached to the outer surface of the front body of disposable diaper 2 at the corresponding side at the waist level.

Referring to Fig. 3, reference numeral 8 designates release tape partially covering the adhesive layer 5 to facilitate the release operation as mentioned above.

Fig. 5 is a sectional view illustrating the essential portion of the second embodiment of the present invention in an enlarged scale. This embodiment is similar to the first embodiment except that the fastener tape 1 comprises first and second portions A, B at opposite ends made of non-elastic body and an intermediate or third portion C made of the base tape 4 consisting only of the base sheet 3 as in the first embodiment.

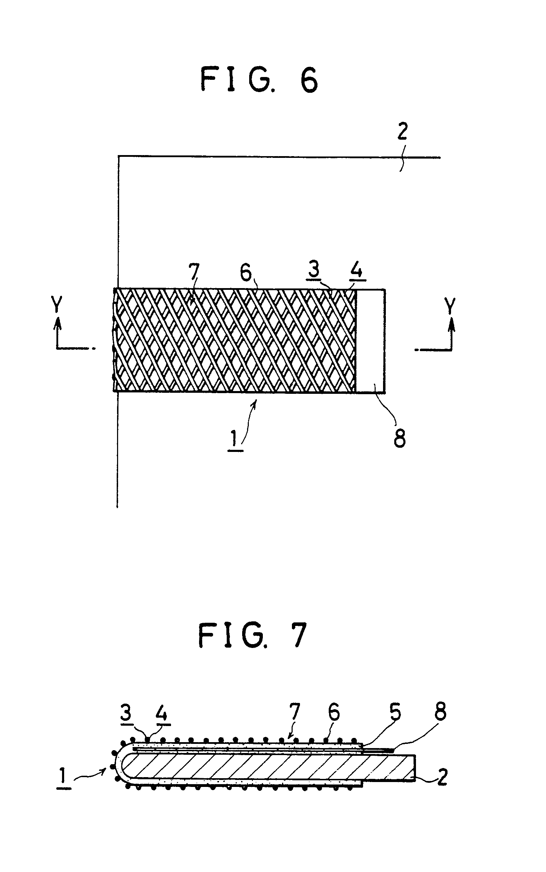

Figs. 6 and 7 are the plan view and the section taken along the line Y - Y in Fig. 6, respectively, illustrating the third embodiment as attached to the rear body of disposable diaper 2 at the waist level on one side. The fastener tape 1 comprises, as in Fig. 3, the base tape 4 consisting only of the base sheet 3 having a hysteresis loss ratio of 20 to 80 % and the adhesive layer 5 covering the entire surface of said base tape 4. However, the base sheet 3 in this embodiment differs from that as shown in Fig. 3 in that comprises a plurality of strands 6 slightly oblique in opposite directions with respect to the width of the fastener tape 1, forming net including a plurality of intersecting points integrated respectively. Areas having no strands 6 define rhombic openings which are longer transversely of the fastener tape 1 and serve as weakened areas 7. Such base sheet 3 and, therefore, the fastener tape 1 are substantially non-elastic transversely of the fastener tape 1 and structurally contractible longitudinally of the fastener tape 1. Also referring to Figs. 6 and 7, reference numeral 8 designates the release tape.

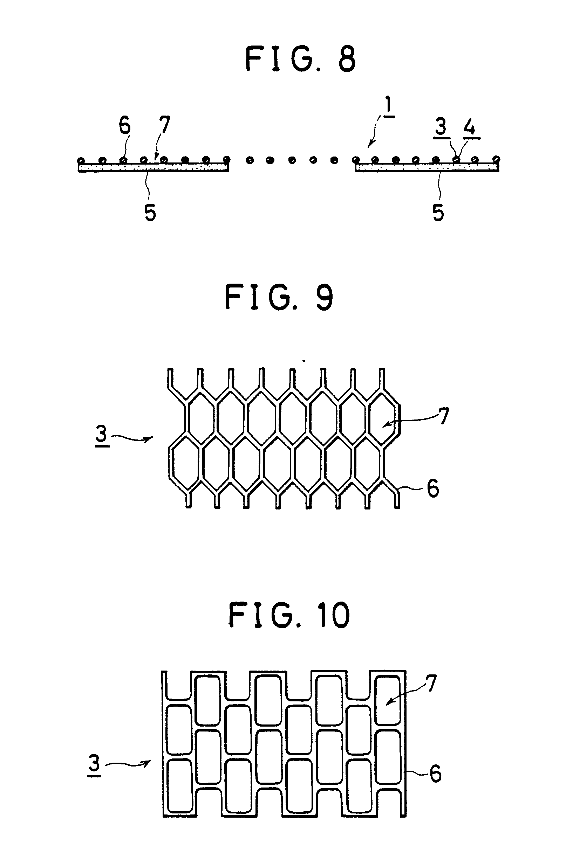

Fig. 8 is the longitudinal section illustrating the fourth embodiment of the present invention, in which the fastener tape 1 is similar to that as shown by Figs. 6 and 7 except that the adhesive layer 5 does not cover the entire surface of the base sheet 3 but the opposite ends only.

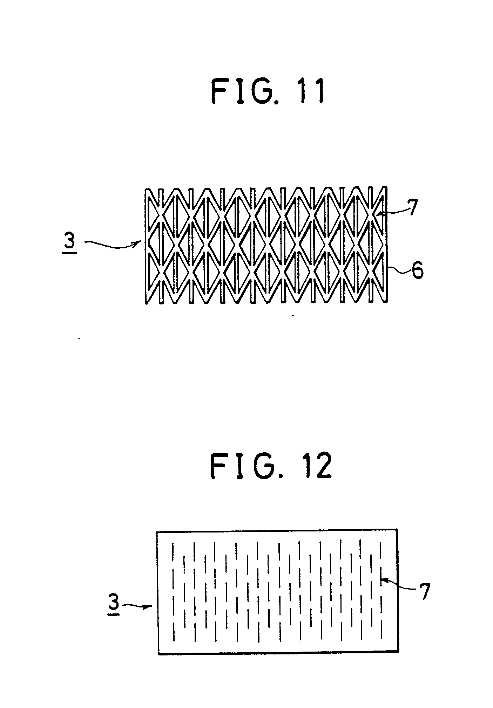

Figs. 9 through 12 are enlarged plan views respectively showing essential portions of the base sheets 3 having the weakened areas 7 of patterns different from that as shown in Fig. 6. Each weakened area 7 is an elongate hexagonal opening in Fig. 9, an elongate, substantially rectangular opening in Fig. 10, an elongate triangular opening in Fig. 11 and an elongate slit in Fig. 12. Any of these base sheets 3 having such weakened areas 7 may be used in the fastener tape according to the present invention. Referring to these figures, reference numeral 6 designates strands.

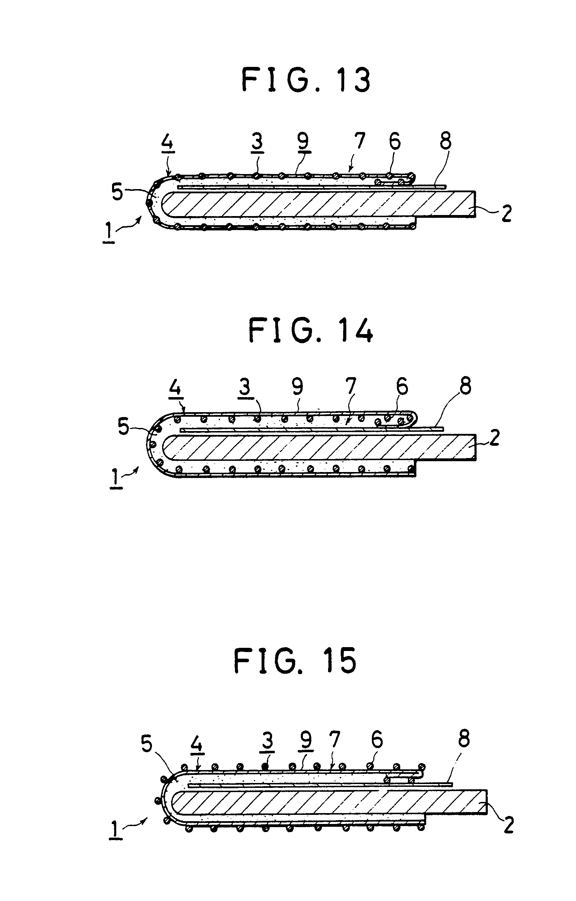

Fig. 13 is a longitudinal section illustrating the fifth embodiment of the present invention as attached to the rear body of disposable diaper 2 at the waist level on one side. The fastener tape 1 comprises the base tape 4 integrally consisting of the base sheet 3 and the elastic body 9, and the adhesive layer 5 covering the entire surface of said base tape 4. Similarly to the embodiment of Fig. 6, the areas having no strands 6 define a plurality of weakened areas 7 in the shape of rhombic openings elongate transversely of the fastener tape 1. The elastic body 9 is integrated with the base sheet 3 so as to close the respective weakened areas 7. The fastener tape 1 is shown to be adhesively attached to the rear body of disposable diaper 2 at the waist level on one side so that the fastener tape 1 is adhesively attached on outer and inner surfaces of said rear body with interposition of the adhesive layer 5. Referring to Fig. 13, reference numeral 8 designates the release tape.

Fig. 14 is the longitudinal section illustrating the sixth embodiment of the present invention, in which the fastener tape 1 is similar to that of Fig. 13 except that the elastic body 9 is disposed on outer side of the base sheet 3.

Fig. 15 is the longitudinal section illustrating the seventh embodiment of the present invention, in which the fastener tape 1 is similar to that of Fig. 13 except that the elastic body 9 is disposed, in contrast with the embodiment of Fig. 14, not on outer side but on inner side of the base sheet 3.

It is obviously possible that the base sheets 3 of Figs. 9 through 12 may be selectively used as the base sheets in the embodiments of Figs. 13 through 15.

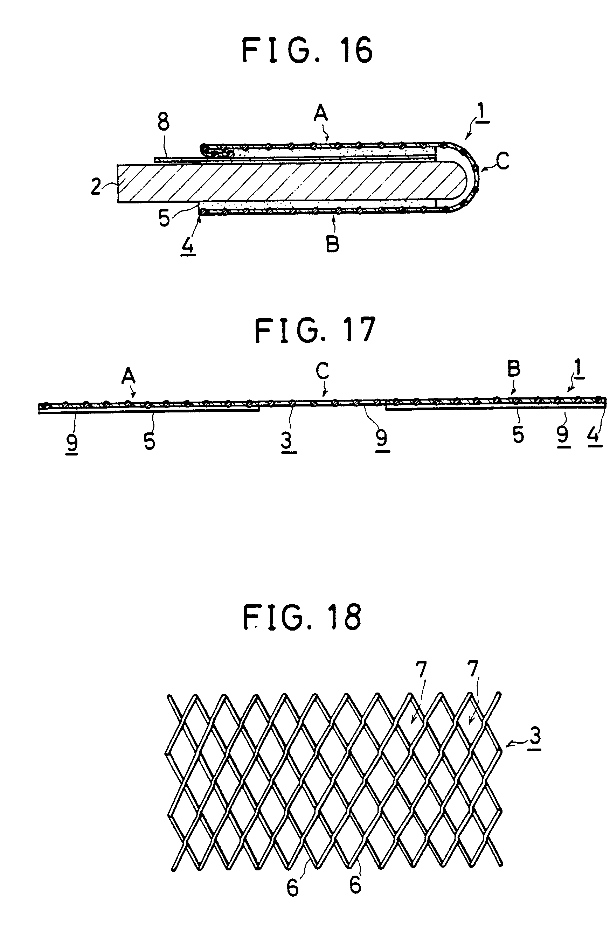

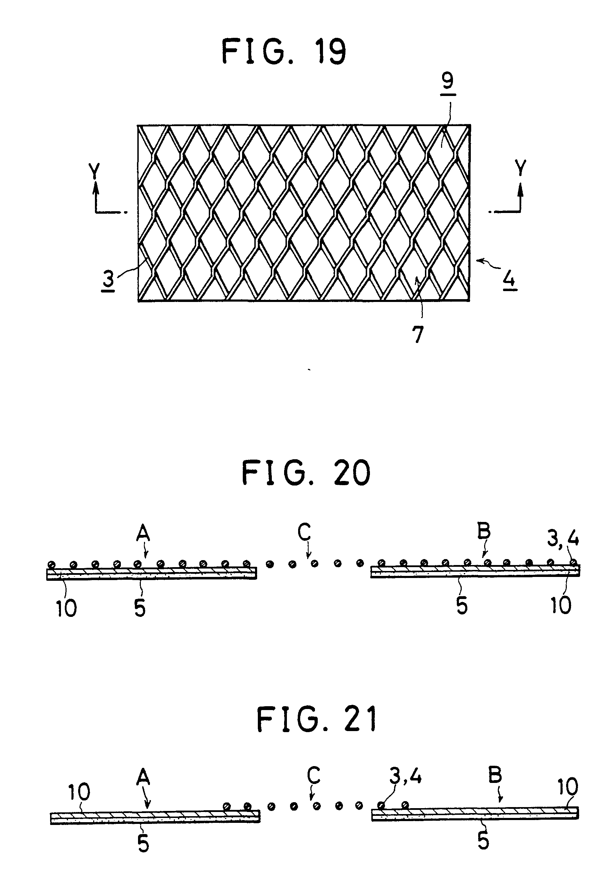

Fig. 16 is the longitudinal section illustrating the eighth embodiment of the present invention as attached to the rear body of disposable diaper 2 at the waist level on one side and Fig. 17 is the longitudinal section illustrating the fastener tape of Fig. 16 prior to attachment. The fastener tape 1 comprises, as that of Fig. 15, the first portion A, the second portion B, which are non-elastic and disposed on the opposite ends, respectively, the intermediate or third portion C having elasticity and the adhesive layer 5 disposed on the surfaces of said first portion A and said second portion B. The first portion A, the second portion B and the third portion C comprise the base tape 4 as shown by Fig. 19 and such base tape 4 consists of the base sheet 3 and the elastic body 9 integrated therewith so as to obtain desired elasticity as will be mentioned later. However, the elastic material 9 is subjected to crosslinking, polymerization or hot calender treatment to make the elastic material nonelastic, so that the elastic material 9 presents elasticity only at the third portin C which has not been subjected to such treatment.

The base sheet 3 in said base tape 4 comprises, as in the embodiment of Fig. 6. a plurality of strands 6 slightly oblique in opposite directions with respect to the width of the fastener tape 1, forming net including a plurality of integrated intersecting points, as shown in Fig. 18, so that the areas having no strands 6 define a plurality of weakened areas 7 in the shape of rhombic openings elongate transversely of the fastener tape 1. Such base sheet 3, as previously pointed out, substantially has no elasticity transversely thereof and is structurally contractible longitudinally.

The elastic material 9 in said base tape 4 is integrated with the base sheet 3 so as to close the respective weakened areas 7, as seen from Figs. 16, 17 and 19. It should be understood that the elastic material 9 may be disposed on the outer or inner surface of the base sheet 3, as seen from Figs. 14 and 15. When it is desired to make the base tape 4 non-elastic, when the base tape 4 is coated with the adhesive layer 5 or when the base tape 4 or the fastener tape 1 is subjected to any secondary processing, it is preferable for operation to shift the base tape 4 or the fastener tape 1 longitudinally of the elongate weakened areas 7, since the base sheet 3 is not substantially deformed in this direction.

It is obvious that said base tape has the hysteresis loss ratio of 20 to 80 %. Also referring to Fig. 16, reference numeral 8 designates the release tape.

Fig. 20 is a longitudinal section illustrating a ninth embodiment of the present invention, in which the fastener tape 1 is similar to the embodiments of Figs 16 through 19 except that the tape 1 is not constructed of the base sheet 3 and the elastic material but consists only of the base sheet 3 and the first portion A and the second portion B comprise non-elastic sheets 10, 10 applied to the base sheet 3 at the opposite ends, respectively.

Fig. 21 is a longitudinal section illustrating the tenth embodiment of the present invention, in which the fastener tape 1 is similar to the embodiment of Fig. 20 except that the base tape 4 consisting only of the base sheet 3 constitutes neither the first portion A nor the second portion B but only the third portion C and nonelastic sheets 10, 10 constituting said first portion A and said second portion B connected to the opposite ends of said base tape 4.

Following experiments and comparative experiments demonstrate the effect of the fastener tape for disposable diaper constructed in accordance with the present invention.

**Experiments 1 - 8 and comparative experiments 1 - 3**

The fastener tape including the base tape consisting of the non-elastic portions and the intermediate elastic portion, as seen in Figs. 5 and 17 is manufactured and attached to the commercially available disposable diaper MERRIES® (Kao Corporation) of L-size. These samples were actually put on 10 monitors (babies weighing 10 kg or higher) for 3 hours and a fitment as well as an oppression around the waist immediately after wearing and immedeately before removal were examined. The result was listed in a Table 1 as shown later.

As the non-elastic portion, oriented polypropyrene film 150 μ thick and as the elastic portion, sheets as mentioned below were used. Width of the base tape was 25 mm and length of the elastic portion was l5 mm.

(1) Sheet used in Experiment 1:

Elastic material (hysteresis loss ratio: 49 %. force required for 200 % stretch: 1300g) consisting of NETLON manufactured by Tokyo Polymer Co. (made of polyethylene of $150g/m^2$, the longitudinal and lateral length of each opening are 2000 μ and 800 μ respectively) applied upon MOBILONFILM© 60 μ thick, polyurethane manufactured by Nisshinbo Co.

(2) Sheet used in Experiment 2:

Elastic material (hysteresis loss ratio: 41 %, force required for 200 % stretch: 1500g) consisting of said NETLON® used in Experiment 1 applied upon said MOBILONFILM® 80 μ thick.

(3) Sheet used in Experiment 3:

Elastic material (hysteresis loss ratio: 32 %, force needed for 120 % stretch: 1050g) consisting of said NETLON® used in Experiment 1 applied upon said MOBILONFILM® 100 μ thick.

(4) Sheet used in Experiment 4:

Elastic material (hysteresis loss ratio: 71 %, force needed for 200 % stretch: 700g) consisting only of said NETLON® used in Experiment 1.

(5) Sheet used in Experiment 5:

Elastic material (hysteresis loss ratio: 60 %, force needed for 100 % stretch: 1100g) consisting only of RABALON ® SJ6400 100 μ thick. i. e., SEBS [polystylenepoly(ethylene-butylene)-polystylene block copolymer] resin manufactured by Mitsubishi Petrochemical Co.

(6) Sheet used in Experiment 6:

Elastic material (hysteresis loss ratio: 41 %, force needed for 200 % stretch: 1300g) consisting only of MILASTOMER 5520B manufactured by Mitsui Petrochemical Industry (polyolefine resin) having a thickness of 250 μ.

(7) Sheet used in Experiment 7:

Elastic material (hysteresis loss ratio: 55 %, force needed for 200 % stretch: 1250g) consisting only of ELASTOMER AR460C (styrene-butadiene copolymer) 300 μ thick manufactured by Alon Kasei Co.

(8) Sheet used in Experiment 8:

Elastic material (hysteresis loss ratio: 35 %, force needed for 200 % stretch: 1180g) consisting only of MITSUI EPT 500 μ thick, styrene-propylene-diene mixture manufactured by Mitsui Petrochemical Industry Co, and exposed four times to electron rays of 5 megarad.

(9) Sheet used in Comparative Experiment 1:

Elastic material (hysteresis loss ratio: 89 %, force needed for 100 % stretch: 200g) consisting only of NETLON® (made of polyethylene of $100g/m^2$, the longitudinal and lateral length of each opening are 1200 μ and 1000 μ respectively).

(10) Sheet used in Comparative Experiment 2:

Elastic material (hysteresis loss ratio: 13 %, force needed for 200 % stretch: 1400g) consisting only of MOBILONFILM® 100 μ thick.

(11) Sheet used in Comparative Experiment 3:

Elastic material (hysteresis loss ratio 12 %, force needed for 100 % stretch: 1350g) consisting of NETLON (made of polyethylen of $70g/m^2$, the longitudinal and lateral length of each opening are 2000 μ and 1800 μ respectively) applied upon MOBILONFILM® (made of polyurethane of $150g/m^2$).

**Table 1**

| | fitment | | oppression around waist |
|---|---|---|---|
| | just after wearing | just before removal | (after use for 3 hours) |
| Experiment 1 | FIG | FIG | substantially no change |
| Experiment 2 | FIG | FIG | slight trace left |
| Experiment 3 | FIG | FIG | slight mark left |
| Experiment 4 | FIG | FIG | no change |
| Experiment 5 | FIG | FIG | no change |

| Experiment 6 | FIG | FIG | slight trace |
| | FIG | FIG | left |
| Experiment 7 | FIG | FIG | substantially no change |
| Experiment 8 | FIG | FIG | slight trace left |
| Comparison Experiment 1 | FIG | FIG | no change |
| Comparison Experiment 2 | FIG | FIG | marked and became reddish |
| Comparison Experiment 3 | FIG | FIG | marked and became reddish |

notes: FIG good
FIG average
FIG below the average
FIG bad

## Claims

1. A fastener tape for disposable diaper comprising a base tape carrying thereon an adhesive layer characterized in that said base tape is elastic; a hysteresis is generated between a tensile force exerted on said base tape and a stretch of said base tape: and a hysteresis loss ratio of a hysteresis curve lies within a range og 20 to 80 %.

2. The fastener tape for disposable diaper according to Claim 1, wherein the base tape comprises a base sheet including a plurality of weakened areas elongate transversely of the sheet and developing substantially no drag and stress in response to any deformation transversely of the sheet.

3. The fastener tape for disposable diaper according to Claim 2, wherein the weakened area are openings.

4. The fastener tape for disposable diaper according to Claim 1, wherein the base tape comprises the base sheet including a plurality of weakened areas elongate transversely of the sheet and developing substantially no drag and stress in response to any deformation transversely of the sheet and an elastic material integrated therewith.

5. The fastener tape for disposable diaper comprising a base tape carrying thereon an adhesive layer: said base tape consisting of a first portion and a second portion both being non-elastic and forming opposite ends of the base tape and an intermediate or third portion having an elasticity characterized in that a hysteresis is generated between a tensile force exerted on said third portion and a stretch of said third portion: and a hysteresis loss ratio of a hysteresis curve lies within a range of 20 to 80 %.

6. The fastener tape for disposable diaper according to Claim 5, wherein the third portion comprises a base sheet including a plurality of weakened areas elongate transversely of the sheet and developing substantially no drag and stress in response to any deformation transversely of the sheet.

7. The fastener tape for disposable diaper according to Claim 5, wherein the adhesive layer is provided only on the surface of the first portion and the second portion.

## Patentansprüche

1. Halteband für Wegwerfwindeln, umfassend ein Grundband mit einer darauf aufgebrachten Klebeschicht, dadurch gekennzeichnet, daß das Grundband elastisch ist, eine Hysterese zwischen einer auf das Grundband ausgeübten Zugkraft und einer Streckung des Grundbands erzeugt wird und ein Hystereseverlustverhältnis einer Hysteresekurve innerhalb eines Bereichs von 20 bis 80 % liegt.

2. Halteband für Wegwerfwindeln nach Anspruch 1, worin das Grundband eine Grundbahn umfaßt, die eine Mehrzahl von schwachen Bereichen, die sich quer zur Bahn erstrecken, einschließt und die im wesentlichen keinen Widerstand und keine Spannung in Bezug auf eine Deformation quer zur Bahn entwickelt.

3. Halteband für Wegwerfwindeln nach Anspruch 1, worin die schwachen Bereiche Öffnungen sind.

4. Halteband für Wegwerfwindeln nach Anspruch 1, worin das Grundband eine Grundbahn, die eine Mehrzahl von schwachen Bereichen, die sich quer zur Bahn erstrecken, einschließt und die im wesentlichen keinen Widerstand und keine spannung in Bezug auf eine Deformation quer zur Bahn entwickelt, und ein damit integriertes elastisches Material umfaßt.

5. Halteband für Wegwerfwindeln, umfassend ein Grundband mit einer darauf aufgebrachten Klebeschicht, wobei das Grundband aus einem ersten Teil und einem zweiten Teil, die beide nichtelastisch sind und die gegenüberliegenden Enden des Grundbands bilden, und einem zwischenteil oder dritten Teil mit einer Elastizität besteht,

dadurch gekennzeichnet,

daß eine Hysterese zwischen einer auf den dritten Teil ausgeübten Zugkraft und einer Streckung des dritten Teils erzeugt wird und ein Hystereseverlustverhältnis einer Hysteresekurve innerhalb eines Bereichs von 20 bis 80 % liegt.

6. Halteband für Wegwerfwindeln nach Anspruch 5, worin der dritte Teil eine Grundbahn umfaßt, die eine Mehrzahl von schwachen Bereichen, die sich quer zur Bahn erstrecken, einschließt und die im wesentlichen keinen Widerstand und keine Spannung in Bezug auf eine Deformation quer zur Bahn entwickelt.

7. Halteband für Wegwerfwindeln nach Anspruch 5, worin die Klebeschicht nur auf der oberfläche des ersten Teils und des zweiten Teils vorgesehen ist.


**Revendications**

1. Bande d'attache pour couche jetable comprenant une bande de base revêtue d'une couche d'adhésif, caractérisée en ce que ladite bande de base est élastique; une hystérésis est produite entre une force de traction exercée sur ladite bande de base et un étirage de ladite bande de base; et un rapport de perte d'une courbe d'hystérésis se situe dans l'intervalle de 20 à 80 %.

2. Bande d'attache pour couche jetable selon la revendication 1, dans laquelle la bande de base comprend une feuille de base comportant une pluralité de zones fragilisées allongées transversalement à la feuille et ne développant pratiquement aucune résistance ni contrainte en réponse à une déformation transversalement à la feuille.

3. Bande d'attache pour couche jetable selon la revendication 2, dans laquelle les zones fragilisées sont des ouvertures.

4. Bande d'attache pour couche jetable selon la revendication 1, dans laquelle la bande de base comprend la feuille de base qui comporte une pluralité de zones fragilisées allongées transversalement à la feuille et ne développant pratiquement ni résistance ni contrainte en réponse à une déformation transversalement à la feuille, et une matière élastique intégrée à celle-ci.

5. Bande d'attache pour couche jetable comprenant une bande de base revêtue d'une couche d'adhésif, ladite bande de base étant constituée d'une première partie et d'une seconde partie toutes deux non élastiques et formant des extrémités opposées de la bande de base, et d'une partie intermédiaire ou troisième partie et présentant une élasticité, caractérisée en ce qu'une hystérésis est produite entre une force de traction exercée sur ladite troisième partie et un étirage de ladite troisième partie; et le rapport de perte d'hystérésis de la courbe d'hystérésis se situe dans l'intervalle de 20 à 80 %.

6. Bande d'attache pour couche jetable selon la revendication 5, dans laquelle la troisième partie comprend une feuille de base comportant une pluralité de zones fragilisées allongées transversalement àa la feuille et ne développant pratiquement ni résistance ni contrainte en réponse à une déformation transversalement à la feuille.

7. Bande d'attache pour couche jetable selon la revendication 5, dans laquelle la couche d'adhésif n'est appliquée que sur la surface de la première partie et de la seconde partie.

# FIG. 1

0                                                          100 %

# FIG. 2

# FIG. 3

# FIG. 4

# FIG. 5

# FIG. 6

# FIG. 7

# FIG. 8

# FIG. 9

# FIG. 10

# FIG. 11

3    7    6

# FIG. 12

3    7

# FIG. 13

# FIG. 14

# FIG. 15

# FIG. 16

# FIG. 17

# FIG. 18

# FIG. 19

# FIG. 20

# FIG. 21